# EUROPEAN PATENT APPLICATION

(11) **EP 4 740 957 A1**
(43) Date of publication of application: **13.05.2026**
(21) Application number: 24835391.4
(22) Date of filing: 04.07.2024
(51) Int. Cl.: A61K 38/48, A61K 31/205, A61K 47/26, A61K 9/08

(54) **BOTULINUM TOXIN PROTEIN COMPOSITION AND USE THEREOF**

(30) Priority: 05.07.2023 CN 202310818072
(71) Applicant: Chongqing Claruvis Pharmaceutical Co., Ltd., Chongqing 400713 (CN)
(72) Inventor: YANG, Wu, Chongqing 400713 (CN); GONG, Hui, Chongqing 400713 (CN)
(74) Representative: HGF
(86) International application number: PCT/CN2024/103534
(87) International publication number: WO 2025/007911

(57) **Abstract**

Provided are a botulinum toxin protein composition and a use thereof, in particular a botulinum toxin protein composition containing L-carnitine or a structural analogue thereof. The botulinum toxin protein composition can be used as a liquid preparation in the fields of medicine and cosmetics. The composition has good stability, particularly long-term stability, and is beneficial to the storage of botulinum toxin proteins.

## Description

### TECHNICAL FIELD

The present disclosure relates to the field of pharmaceutical formulations, and in particular, to a botulinum toxin protein composition and use thereof, particularly a botulinum toxin protein composition comprising L-carnitine or a structural analog thereof.

### BACKGROUND

Native botulinum neurotoxins (BoNTs) are toxin proteins produced by an anaerobic bacterium *Clostridium botulinum.* They cause muscle paralysis in mammals by blocking the presynaptic release of neurotransmitter acetylcholine at the neuromuscular junction. Currently, seven types of BoNTs have been identified, designated as BoNT/A through BoNT/G, respectively. BoNTs have rapidly become therapies for cholinergic hyperexcitability due to their unique pharmacological properties including high specificity for the nervous system, limited diffusion by local injection, and reversible effects, and possess broad applications in the fields of medicine and cosmetology.

In general, therapeutically and pharmaceutically active proteins are prepared in solutions, in particular for injections. Therapeutically active proteins are generally prepared as compositions and commercialized as a ready-to-use solution or a lyophilized form for reconstitution into a solution. For example, commercial BoNT/A products are vacuum-dried or lyophilized solid formulations and require reconstitution by healthcare providers at the time of use, thus requiring precise control of the reconstitution volume. In addition, due to the differences in therapeutic purpose and individual dosage between patients, the volume required for each dose varies considerably. After reconstituting the commercial products to the volume specified by the manufacturer, clinicians often administer only a small portion of the content in the vial to the patient, and preserve the remainder in a refrigerator for later use. It has been estimated that the preservation of a reconstituted BoNT/A formulation in a refrigerator (about 4 °C) for 12 h may lead to a 44% or greater loss of efficacy. Thus in practice, it is generally recommended to use the product within 4 h after reconstitution, which results in a waste of medications and an unnecessary economic burden for the patient. In addition, due to the extremely low concentration of botulinum toxin in use, it is generally prepared with the addition of high concentrations of exogenous proteins to keep the stability of the botulinum toxin protein at the low concentration and avoid the adsorption of the active ingredient onto solid surfaces. In most botulinum toxin formulations, albumin or gelatin is used as the stabilizer, which not only causes immune responses, but also has the potential to introduce a source of contamination.

### SUMMARY

To overcome the defects in the prior art, the present disclosure provides a botulinum toxin protein composition, a preparation method therefor, and use thereof.

In a first aspect of the present disclosure, provided is a botulinum toxin protein composition comprising a botulinum toxin protein, and L-carnitine or a derivative thereof or a salt thereof.

In one embodiment of the present disclosure, the composition is a liquid composition further comprising a solvent, such as water (particularly water for injection) and sodium chloride injection (e.g., 0.9% sodium chloride injection and compound sodium chloride injection).

Specifically, the solvent is a sterile solvent, such as sterilized water for injection and 0.9% sodium chloride injection.

Specifically, the L-carnitine or the derivative thereof or the salt thereof in the composition has a concentration of 1 mM to 50 mM (e.g., 1 mM, 2 mM, 3 mM, 4 mM, 4.5 mM, 4.8 mM, 4.9 mM, 5 mM, 6 mM, 8 mM, 9 mM, 10 mM, 11 mM, 12 mM, 14 mM, 15 mM, 20 mM, 20.1 mM, 20.2 mM, 20.5 mM, 25 mM, 30 mM, 35 mM, 40 mM, 45 mM, or 50 mM), such as 1 mM to 4.9 mM, 2 mM to 4.5 mM, 1 mM to 20 mM, 20.1 mM to 50 mM, 20.5 mM to 40 mM, or 20.5 mM to 30 mM.

In one embodiment of the present disclosure, the composition comprises botulinum toxin protein and L-camitine.

In one embodiment of the present disclosure, the composition comprises the botulinum toxin protein and the salt of the L-camitine.

In one embodiment of the present disclosure, the composition comprises the botulinum toxin protein and the derivative of the L-carnitine or the salt thereof.

In the composition, the L-carnitine or the derivative thereof or the salt thereof is used as a protein stabilizer; in one embodiment, the protein stabilizer is the L-camitine; in another embodiment, the protein stabilizer is the salt of the L-camitine; in another embodiment, the protein stabilizer is the derivative of the L-camitine; in another embodiment, the protein stabilizer is the salt of the derivative of the L-camitine.

In some embodiments of the present disclosure, the derivative of the L-carnitine is alkanoyl L-carnitine having a structural formula of 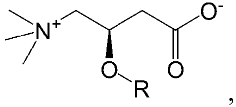 wherein R represents alkanoyl, which may be saturated or unsaturated and linear or branched, and has 2 to 26 carbon atoms (e.g., 2, 3, 4, 5, 6, 7, 8, 9, 10, 11, 12, 14, 16, 18, 20, 22, 24, or 26 carbon atoms), such as acetyl, propionyl, butyryl, isobutyryl, valeryl, or isovaleryl. In one embodiment of the present disclosure, the alkanoyl L-carnitine is O-acetyl-L-carnitine.

Specifically, the salt of the L-carnitine or the derivative thereof is preferably a pharmaceutically acceptable salt, which is any salt of the L-carnitine or the derivative thereof with an acid that does not produce toxic and side effects, comprising, but not limited to, salts formed with inorganic acids such as hydrochloride, nitrate, phosphate, sulfate, hydrobromide, hydroiodide; salts formed with inorganic acids such as aliphatic mono-carboxylic acids and dicarboxylic acids, phenyl-substituted alkanoic acids, hydroxyalkanoic acids, alkanedioic acids, aromatic acids, and aliphatic and aromatic sulfonic acids, for example, sulfate, pyrosulfate, bisulfate, sulfite, bisulfite, nitrate, phosphate, monohydrogen phosphate, dihydrogen phosphate, metaphosphate, pyrophosphate, hydrochloride, hydrobromide, iodate, acetate, propionate, caprylate, isobutyrate, oxalate, malonate, succinate, suberate, sebacate, fumarate, maleate, mandelate, benzoate, chlorobenzoate, methylbenzoate, dinitrobenzoate, phthalate, benzenesulfonate, tosylate, phenylacetate, citrate, lactate, tartrate, methanesulfonate, gluconate, galacturonate, mucate, glucose phosphate, trichloroacetate, trifluoroacetate, aspartate, fumarate, glycerophosphate, methanesulfonate, and choline bitartrate. In some embodiments of the present disclosure, the salt is tartrate, hydrochloride, citrate, succinate, or fumarate.

In one embodiment of the present disclosure, the salt is hydrochloride.

In one embodiment of the present disclosure, the salt is tartrate.

In general, proteins are highly susceptible to degradation and inactivation at low concentrations (< 0.1 mg/mL). In the present disclosure, it has been found that L-carnitine or a derivative thereof (e.g., alkanoyl L-carnitine) or a salt thereof (e.g., tartrate or hydrochloride) can provide the formulation with the characteristic of low viscosity, and can reduce the aggregation and precipitation of the botulinum toxin protein, which is beneficial to maintaining the stability of the botulinum toxin protein.

Specifically, the botulinum toxin protein may be selected from any one of botulinum toxin protein type A, botulinum toxin protein type B, botulinum toxin protein type C1, botulinum toxin protein type C2, botulinum toxin protein type D, botulinum toxin protein type E, botulinum toxin protein type F, or botulinum toxin protein type G, and is particularly botulinum toxin protein type A.

In one embodiment of the present disclosure, the botulinum toxin is a recombinant botulinum toxin, particularly recombinant botulinum toxin protein type A, such as the toxin polypeptide described in WO2022/179552A1, particularly the dimeric BoNT/A prepared in the examples thereof.

Specifically, the botulinum toxin protein in the composition may be at a concentration of 1 U/mL to 1000 U/mL (e.g., 1 U/mL, 10 U/mL, 50 U/mL, 60 U/mL, 70 U/mL, 80 U/mL, 90 U/mL, 100 U/mL, 150 U/mL, 200 U/mL, 250 U/mL, 300 U/mL, 400 U/mL, 500 U/mL, 600 U/mL, 700 U/mL, 800 U/mL, 900 U/mL, or 1000 U/mL), particularly 50 U/mL to 200 U/mL.

In one embodiment of the present disclosure, the composition further comprises a solubilizer.

Specifically, in the composition, the volume percentage concentration of the solubilizer is 0% to 0.1% (e.g., 0.01%, 0.02%, 0.05%, or 0.1%), such as 0.01% to 0.1%.

Specifically, the solubilizer is selected from one or more of a Tween, a Span, sodium dodecyl sulfate, and poloxamer.

More specifically, the Tween solubilizer is selected from one or more of Tween 20, Tween 40, Tween 60, or Tween 80.

More specifically, the Span solubilizer is selected from one or more of Span 20, Span 40, Span 60, Span 80, or Span 85.

In some embodiments of the present disclosure, the solubilizer is Tween 80.

In one embodiment of the present disclosure, the composition is a liquid composition comprising water (particularly water for injection) as a solvent, and the composition further comprises an osmotic pressure regulator.

Specifically, the osmotic pressure regulator is selected from sodium chloride, glucose, glycerol, fructose, sorbitol, magnesium chloride, phosphate, sodium citrate, and mannitol, and is particularly sodium chloride.

Specifically, in the composition, the mass percentage concentration of the osmotic pressure regulator is 0.6% to 0.9% (e.g., 0.6%, 0.7%, 0.8%, or 0.9%); preferably, the osmotic pressure regulator is an isotonic regulator, the amount of which is determined so as to adjust the osmotic pressure of the composition to be equal or close to the osmotic pressure of plasma.

In some embodiments of the present disclosure, the osmotic pressure regulator is sodium chloride at a mass percentage concentration of 0.9% in the composition.

Specifically, the composition may comprise: the botulinum toxin protein, a protein stabilizer (the L-carnitine or the derivative thereof or the salt thereof), the solubilizer, and the osmotic pressure regulator, wherein the L-carnitine or the derivative thereof or the salt thereof has a concentration of 1 mM to 50 mM, the solubilizer has a volume percentage concentration of 0% to 0.1%, and the osmotic pressure regulator is an isotonic regulator; the selection of each component is as described above in the present disclosure.

More specifically, the composition comprises the botulinum toxin protein, 2 mM to 10 mM of the protein stabilizer (the L-carnitine or the derivative thereof or the salt thereof), 0% to 0.1% Tween 80, and 0.9% sodium chloride.

In some embodiments of the present disclosure, the composition comprises the botulinum toxin protein, 10 mM of the protein stabilizer (the L-carnitine or the derivative thereof or the salt thereof), 0.1% Tween 80, and 0.9% sodium chloride.

Specifically, the composition has a pH of 5.0 to 8.0 (e.g., 5.0, 5.5, 6.0, 6.5, 7.0, 7.5, or 8.0).

In one embodiment of the present disclosure, the composition further comprises a second (cosmetic) active ingredient, such as one or more of hyaluronic acid (hyaluronate) or a salt thereof, polylactic acid, polycaprolactone, and collagen, so as to achieve a combined effect (e.g., shrinking pores and lightening skin tone).

In one embodiment of the present disclosure, the composition further comprises a local anesthetic, such as lidocaine, bupivacaine, ropivacaine, and tetracaine.

Specifically, the composition may further comprise one or more excipients such as a cosolvent, a pH regulator, an antioxidant, and a buffer.

In a second aspect of the present disclosure, provided is a preparation method for the composition according to the first aspect. The method comprises a step of mixing the components.

In one embodiment of the present disclosure, the composition is a liquid composition further comprising a solvent, and the preparation method comprises the following steps:
(1) mixing a solvent and L-carnitine or a derivative thereof or a salt thereof (optionally, a solubilizer, and, if necessary, other components);
(2) diluting a botulinum toxin protein with the mixture obtained in step (1); and optionally, (3) aliquoting and storing the mixture obtained in step (2).

In some embodiments of the present disclosure, the solvent is 0.9% sodium chloride injection, and step (1) comprises: mixing the solvent, L-carnitine or the derivative thereof or the salt thereof, and the solubilizer.

Specifically, the dilution in step (2) may be performed once or by multiple consecutive dilutions to the desired concentration.

Specifically, the aliquoting in step (3) may be performed by using vials.

Specifically, the storage temperature in step (3) is a low temperature, particularly 2 °C to 8 °C (e.g., 2 °C, 4 °C, 6 °C, or 8 °C).

In a third aspect of the present disclosure, provided is use of the composition according to the first aspect in preparing a medicament for preventing and/or treating a disease.

In one embodiment of the present disclosure, the disease is associated with hyperactive neurons or glands, such as neuromuscular diseases, autonomic disorders, and pain.

More specifically, the disease comprises spasmodic dysphonia, spasmodic torticollis, laryngeal dystonia, oromandibular dystonia, lingual dystonia, cervical dystonia, focal dystonia, blepharospasm, strabismus, hemifacial spasm, eyelid disorder, cerebral palsy, focal spasm and other speech disorders, spastic colitis, neurogenic bladder, anismus, limb spasm, tics, tremor, bruxism, anal fissure, achalasia, dysphagia and other dystonias as well as other disorders characterized by muscle group involuntary movement, lacrimation, hyperhidrosis, excessive salivation, excessive gastrointestinal secretion, secretory disorders, pain due to muscle spasm, headache, and the like.

In one embodiment of the present disclosure, the disease is not associated with undesired neuronal activity, such as psoriasis, allergy, hemophagocytic lymphohistiocytosis, and alcoholic pancreatic disease.

In one embodiment of the present disclosure, the disease is a thalamus-mediated disorder, such as epilepsy or chronic pain.

More specifically, the chronic pain is selected from central sensitization chronic pain, post-stroke central pain, regional pain, phantom limb pain, and demyelinating pain.

In one embodiment of the present disclosure, the disease is neuropsychiatric disorders, such as schizophrenia, delirium, Alzheimer's disease, depression, mania, attention deficit disorder, drug addiction, dementia, agitation, apathy, anxiety, psychosis, personality disorder, bipolar disorder, obsessive compulsive disorder, eating disorder, post-traumatic stress disorder, irritability, and disinhibition, particularly schizophrenia, Alzheimer's disease, mania, anxiety disorder, and depression.

Specifically, the medicament may be an injection.

In a fourth aspect of the present disclosure, provided is use of the composition according to the first aspect in preparing a cosmetic product.

Specifically, the cosmetic use comprises wrinkle removal or prevention, facial slimming, scar repair, improvement or removal of pimples or acne, eyebrow shape modification, mouth corner lifting, body contouring (e.g., slimming legs or shoulders), body odor removal, hair growth, hair retention, and the like, particularly wrinkle removal and facial slimming.

Specifically, the wrinkles may be viewed as folds, ridges, or creases in the skin. Wrinkles visible in the human face are preferred. The wrinkles comprise, but are not limited to, tear troughs, glabellar lines, crow's feet, buccal commissure lines, mandibular lines, perioral wrinkles, buccal folds, marionette lines, lip lines, forehead wrinkles, frown lines, bunny lines, nasolabial folds, infraocular wrinkles, chin folds, neck lines, and the like, particularly glabellar lines, crow's feet, and forehead wrinkles.

Specifically, the product may be an injection.

In a fifth aspect of the present disclosure, provided is a method for preventing and/or treating a disease. The method comprises a step of administering to a subject in need thereof an effective amount of the composition according to the first aspect.

Specifically, the disease is as described in the third aspect of the present disclosure.

Specifically, the subject is a mammal, particularly a human.

Specifically, the mode of administration is injection administration, such as subcutaneous injection, intradermal injection, or intramuscular injection.

In a sixth aspect of the present disclosure, provided is a cosmetic method. The method comprises a step of administering to a subject in need thereof an effective amount of the composition according to the first aspect.

Specifically, the cosmetic use comprises wrinkle removal or prevention, facial slimming, scar repair, improvement or removal of pimples or acne, eyebrow shape modification, mouth corner lifting, body contouring (e.g., slimming legs or shoulders), body odor removal, hair growth, hair retention, and the like, particularly wrinkle removal and facial slimming.

Specifically, the subject is a mammal, particularly a human.

Specifically, the mode of administration is injection, such as subcutaneous injection, intradermal injection, or intramuscular injection.

Compared with the prior art, the present disclosure has the following beneficial effects: The present disclosure provides a stable botulinum toxin protein composition in which L-carnitine or a derivative thereof or a salt thereof is used as a protein stabilizer to replace human-derived protein (e.g., human serum albumin), thereby eliminating the threat of human-derived viruses. Meanwhile, in combination with Tween 80, the composition can effectively prevent adsorption and achieve better long-term stability and can even maintain high protein activity for more than two years. Furthermore, since the botulinum toxin protein composition is prepared as a liquid formulation, the preparation method is simpler, and the cost can be effectively reduced, which is suitable for industrial-scale production.

When used as a liquid formulation, the botulinum toxin protein composition of the present disclosure increases convenience in clinical use and addresses the common shortcomings of lyophilized or vacuum-processed products, such as short shelf lives after reconstitution, susceptibility to waste, human-caused contaminations during the reconstitution process, inaccurate reconstitution, and the like.

### DETAILED DESCRIPTION

Unless otherwise defined, all scientific and technical terms used in the present disclosure have the same meaning as commonly understood by those skilled in the art to which the present disclosure relates.

It has been reported that the use of botulinum toxin to treat various spasmodic muscular disorders can reduce depression and anxiety while alleviating muscle spasm. Murry T., et al., Spasmodic dysphonia; emotional status and botulinum toxin treatment, Arch Otolaryngol 1994 Mar; 120(3): 310-316; Jahanshahi M., et al., Psychological functioning before and after treatment of torticollis with botulinum toxin, J Neurol Neurosurg Psychiatry 1992; 55(3): 229-231. In addition, the German patent application DE10150415 discusses the intramuscular injection of botulinum toxin for the treatment of depression and related affective disorders.

Botulinum toxin is further proposed for or has already been used for the treatment of skin lesions (U.S. Patent No. 6,447,787), various autonomic dysfunctions (U.S. Patent No. 5,766,605), tension headache (U.S. Patent No. 6,458,365), migraine (U.S. Patent No. 5,714,468), sinus headache (U.S. Patent Application No. 429069), post-operative pain and visceral pain (U.S. Patent No. 6,464,986), neuralgia (U.S. Patent Application No. 630,587), hair growth and hair retention (U.S. Patent No. 6,299,893), dental diseases (U.S. Provisional Patent Application No. 60/418,789), fibromyalgia (U.S. Patent No. 6,623,742), various skin diseases (U.S. Patent Application No. 10/731973), motion sickness (U.S. Patent No. 752,869), psoriasis and dermatitis (U.S. Patent No. 5,670,484), injured muscles (U.S. Patent No. 6,423,319), various cancers (U.S. Patent No. 6,139,845), smooth muscle disorders (U.S. Patent No. 5,437,291), downturn at corners of the mouth (U.S. Patent No. 6,358,917), nerve entrapment syndromes (U.S. Patent Application No. 20030224019), various impulse disorders (U.S. Patent Application No. 423,380), acne (WO03/011333), and neurogenic inflammation (U.S. Patent No. 6,063,768).

In the present disclosure, the term "U" refers to a potency unit, a unit used in pharmaceutics representing the diversity of biological abilities. Since the chemical composition of certain pharmaceuticals is not constant or their quality specifications cannot yet be determined by physicochemical methods, the potency of such pharmaceuticals is often determined by biological assays and comparisons with standards. By this bioassay, the smallest potency unit with a certain biological potency is called the "unit" (U); a standard unit specified via international negotiation is called the "international unit" (IU).

The term "tag protein" refers to a polypeptide or protein that is fused to and expressed with a target protein using *in vitro* DNA recombinant technology, so as to facilitate the expression and detection of the target protein.

The term "dimeric structure" refers to a macromolecular complex composed of two molecules, often bonded by non-covalent bonds.

The terms "levocamitine" and "L-camitine" represent the same substance and may be used interchangeably.

The disclosures of the various publications, patents, and published patent specifications cited herein are hereby incorporated by reference in their entireties.

The technical solutions of the present disclosure will be clearly and completely described below with reference to the examples of the present disclosure, and it is obvious that the described examples are only a part of the examples of the present disclosure but not all of them. Based on the examples of the present disclosure, all other examples obtained by those of ordinary skill in the art without creative work shall fall within the protection scope of the present disclosure.

The detection method for the amount of the botulinum toxin protein used in the examples is as follows:
The activity of the botulinum toxin protein was assessed by the median lethal dose of mice and expressed in mouse units (U). One unit is defined as the amount of the botulinum toxin protein required for intraperitoneal injection to kill 50% of the mice.

### Animal study procedures:

### 1. Species and grouping:

Adult CD-1 mice were selected, grouped, weighed, and identified to ensure that each group had an equal number of mice with similar body weights.

### 2. Administration:

0.5 mL of botulinum toxin protein formulations of different compositions (diluted if necessary) was administrated to the CD-1 mice via a single intraperitoneal injection.

### 3. Observation:

Before the start of the study, the body weight of each mouse was measured once (before the administration ) and recorded. After the administration, each animal was weighed every 24 h, and the mortality was recorded at 24 h (±1 h), 48 h (±1 h), 72 h (±1 h), and 96 h (±1 h) post-dose. The 95% confidence limit should be within 50% to 200% of the potency.

The recombinant botulinum toxin protein type A used in the examples was prepared with reference to the method described in Examples 1 to 6 of WO2022/179552A1, i.e., dimeric BoNT/A.

### Example 1

Formulations were prepared according to the formulas shown in Table 1. The botulinum toxin was recombinant botulinum toxin protein type A, and the content of the recombinant botulinum toxin protein type A in all the formulations was 100 U/mL. Taking formula 1 in Table 1 as an example, the preparation procedures were as follows:
1. Preparation of solvent (100 mL):
   a) pipetting 99 mL of 0.9% sodium chloride injection into a solution bottle;
   b) adding 0.202 g of L-carnitine hydrochloride and 0.1 mL of Tween 80 into the solution bottle;
   c) mixing uniformly at 100 rpm for 15 min for later use.
2. Dilution of recombinant botulinum toxin protein type A formulation:
   a) first-step dilution with a dilution factor of 100-fold;
   b) second-step dilution with a dilution factor of 100-fold;
   c) third-step dilution to 100 U/mL;
   d) aliquoting into vials, labeling, and subsequently storing at a preset temperature (2 °C to 8 °C).

Samples were taken at different time points to detect the botulinum toxin activity of the resulting formulations. The results are shown in Table 1.

**Table 1. Formulas and long-term stability detection results**

| Formula No. | Composition | 0 days after formulation | 60 days after formulation |
|---|---|---|---|
| 1 | 0.1% Tween 80, 10 mM L-carnitine hydrochloride, and 0.9% sodium chloride | 92 U/ml | 93 U/ml |
| 2 | 0.1% Tween 80, 10 mM L-carnitine tartrate, and 0.9% sodium chloride | 66 U/ml | 113 U/ml |

| | | | |
|---|---|---|---|
| Note: The protein activity for formula 2 was greater than 100 U/mL 60 days after formulation, which was a conventional fluctuation and complied with the requirements for accuracy and precision of test methods involving animal experiments in the General Principles for Technical Review of Analytical Method Validation for Biological Products issued by the China Center for Drug Evaluation. The same applies to other examples. | | | |

As shown in Table 1, the formulations comprising the L-carnitine salt exhibited better stability and could effectively maintain protein activity for 60 days.

### Example 2

Formulations were prepared according to the formulas shown in Table 2 with reference to the preparation procedures in Example 1. The botulinum toxin was recombinant botulinum toxin protein type A, and the content of the recombinant botulinum toxin protein type A in all the formulations was 100 U/mL.

After the preparation was completed, the botulinum toxin activity of the resulting formulations was detected. The results are shown in Table 2.

**Table 2. Formulas and activity detection results**

| Formula No. | Composition | Activity |
|---|---|---|
| 1 | 0.1% Tween 80, 10 mM O-acetyl-L-carnitine hydrochloride, and 0.9% sodium chloride | 92 U/ml |
| 2 | 0.1% Tween 80, 10 mM L-carnitine hydrochloride, and 0.9% sodium chloride | 92 U/ml |

As shown in Table 2, alkanoyl L-carnitine could also serve as a protein stabilizer and exert the same effect as L-camitine.

### Example 3

Formulations were prepared according to the formulas shown in Table 3 with reference to the preparation procedures in Example 1. The botulinum toxin was recombinant botulinum toxin protein type A, and the content of the recombinant botulinum toxin protein type A in all the formulations was 350 U/mL.

After the preparation was completed, the botulinum toxin activity of the resulting formulations was detected. The results are shown in Table 3.

**Table 3. Formulas and activity detection results**

| Formula No. | Composition | Activity |
|---|---|---|
| 1 | 10 mM L-carnitine, 0.01% Tween 80, and 0.9% sodium chloride | 339 U/ml |
| 2 | 10 mM L-carnitine, 0.02% Tween 80, and 0.9% sodium chloride | 400 U/ml |
| 3 | 10 mM L-camitine, 0.05% Tween 80, and 0.9% sodium chloride | 357 U/ml |
| 4 | 10 mM L-camitine, 0.1% Tween 80, and 0.9% sodium chloride | 359 U/ml |

As shown in Table 3, Tween at the concentration range of 0.01% to 0.1% could effectively maintain the botulinum toxin activity.

### Example 4

Formulations were prepared according to the formulas shown in Table 4 with reference to the preparation procedures in Example 1. The botulinum toxin was recombinant botulinum toxin protein type A, and the content of the recombinant botulinum toxin protein type A in all the formulations was 200 U/mL.

After the preparation was completed, the botulinum toxin activity of the resulting formulations was detected. The results are shown in Table 4.

**Table 4. Formulas and activity detection results**

| Formula No. | Composition | Activity |
|---|---|---|
| 1 | 2 mM L-camitine, 0.02% Tween 80, and 0.9% sodium chloride | 299 U/ml |
| 2 | 2 mM L-camitine, 0.05% Tween 80, and 0.9% sodium chloride | 236 U/ml |
| 3 | 2 mM L-camitine, 0.1% Tween 80, and 0.9% sodium chloride | 228 U/ml |
| 4 | 10 mM L-camitine, 0.02% Tween 80, and 0.9% sodium chloride | 277 U/ml |
| 5 | 10 mM L-camitine, 0.05% Tween 80, and 0.9% sodium chloride | 235 U/ml |
| 6 | 10 mM L-camitine, 0.1% Tween 80, and 0.9% sodium chloride | 222 U/ml |

As shown in Table 4, all the formulations comprising 2 mM to 10 mM levocamitine could effectively maintain the botulinum toxin activity.

### Example 5

Formulations were prepared according to the formulas shown in Table 5 with reference to the preparation procedures in Example 1. The botulinum toxin was recombinant botulinum toxin protein type A, and the content of the recombinant botulinum toxin protein type A in all the formulations was 100 U/mL.

Samples were taken at different time points to detect the botulinum toxin activity of the resulting formulations, so as to investigate the long-term stability of the formulations. The results are shown in Table 5.

**Table 5. Formulas and long-term stability detection results**

| Formula No. | Composition | 44 days | 70 days | 520 days | 733 days |
|---|---|---|---|---|---|
| 1 | 0.1% Tween 80, 10 mM levocamitine, and 0.9% sodium chloride | 107 U/ml | 104 U/ml | 86 U/ml | 71U/ml |
| 2 | 0.1% Tween 80, 10 mM L-histidine, and 0.9% sodium chloride | 86 U/ml | 87 U/ml | 82 U/ml | N/A |

As shown in Table 5, formulations comprising levocamitine exhibited excellent stability over a long period of time and could maintain high activity for more than two years.

The above description is only for the purpose of illustrating the preferred examples of the present disclosure, and is not intended to limit the scope of the present disclosure. Any modifications, equivalent substitutions, and the like made without departing from the spirit and principle of the present disclosure shall fall within the protection scope of the present disclosure.

The foregoing examples and methods described herein may vary based on the abilities, experience, and preferences of those skilled in the art.

The certain order in which the steps of the method are listed in the present disclosure does not constitute any limitation on the order of the steps of the method.

## Claims

1. A botulinum toxin protein composition, comprising: a botulinum toxin protein and L-carnitine or a derivative thereof or a salt of L-carnitine or of the derivative thereof, wherein preferably, the composition is a liquid composition, and more preferably, the composition further comprises a solvent, such as water for injection or 0.9% sodium chloride injection.

2. The composition according to claim 1, wherein the L-carnitine or the derivative thereof or the salt thereof has a concentration of 1 mM to 50 mM, preferably 1 mM to 20 mM.

3. The composition according to claim 1 or 2, wherein the derivative of the L-carnitine is alkanoyl L-camitine;
preferably, the alkanoyl group has 2 to 26 carbon atoms;
more preferably, the alkanoyl is acetyl, propionyl, butyryl, isobutyryl, valeryl or isovaleryl;
more preferably, the derivative of the L-carnitine is O-acetyl-L-carnitine.

4. The composition according to claim 1 or 2, wherein the salt of the L-carnitine or the derivative thereof is a pharmaceutically acceptable salt, preferably selected from tartrate, hydrochloride, citrate, succinate and fumarate, more preferably selected from tartrate or hydrochloride.

5. The composition according to claim 1, further comprising a solubilizer,
wherein preferably, a volume percentage concentration of the solubilizer is 0% to 0.1%; preferably, the solubilizer is selected from one or more of a Tween, a Span, sodium dodecyl sulfate and poloxamer;
more preferably, the solubilizer is Tween 80.

6. The composition according to claim 1, further comprising an osmotic pressure regulator,
wherein preferably, the osmotic pressure regulator is selected from sodium chloride, glucose, glycerol, fructose, sorbitol, magnesium chloride, phosphate, sodium citrate and mannitol;
more preferably, the osmotic pressure regulator is sodium chloride at a mass percentage concentration of 0.9% in the composition.

7. The composition according to any one of claims 1 to 6, wherein the botulinum toxin protein is selected from any one of botulinum toxin protein type A, botulinum toxin protein type B, botulinum toxin protein type C1, botulinum toxin protein type C2, botulinum toxin protein type D, botulinum toxin protein type E, botulinum toxin protein type F or botulinum toxin protein type G, and is preferably botulinum toxin protein type A;
preferably, the botulinum toxin is a recombinant botulinum toxin.

8. The composition according to claim 7, comprising the botulinum toxin protein, the L-carnitine or the derivative thereof or a salt of L-carnitine or of the derivative thereof, the solubilizer, and the osmotic pressure regulator, wherein the L-carnitine or the derivative thereof or the salt thereof has a concentration of 1 mM to 50 mM, the solubilizer has a volume percentage concentration of 0% to 0.1%, and the osmotic pressure regulator is an isotonic regulator;
preferably, the composition comprises the botulinum toxin protein, 2 mM to 10 mM of the L-carnitine or the derivative thereof or the salt thereof, 0% to 0.1% Tween 80, and 0.9% sodium chloride;
more preferably, the composition comprises the botulinum toxin protein, 10 mM of the L-carnitine or the derivative thereof or the salt thereof, 0.1% Tween 80, and 0.9% sodium chloride.

9. Use of the composition according to any one of claims 1 to 8 in preparing a medicament for preventing and/or treating a disease and a cosmetic product.

10. The use according to claim 9, wherein the disease is selected from neuromuscular diseases, autonomic disorders, pain, neuropsychiatric disorders, skin diseases, dental diseases and cancer;
preferably, the disease is selected from spasmodic dysphonia, spasmodic torticollis, laryngeal dystonia, oromandibular dysphonia, lingual dystonia, cervical dystonia, focal dystonia, blepharospasm, strabismus, hemifacial spasm, eyelid disorder, cerebral palsy, focal spasm and other language disorders, spastic colitis, neurogenic bladder, anismus, limb spasm, tics, tremor, bruxism, anal fissure, achalasia, dysphagia and other dystonias as well as other disorders **characterized by** muscle group involuntary movement, lacrimation, hyperhidrosis, excessive salivation, excessive gastrointestinal secretion, secretory disorders, pain due to muscle spasm, headache; psoriasis, allergy, hemophagocytic lymphohistiocytosis and alcoholic pancreatic disease; epilepsy, central sensitization chronic pain, post-stroke central pain, regional pain, phantom limb pain and demyelinating pain; schizophrenia, Alzheimer's disease, mania, anxiety disorder, depression; tension headache, migraine, sinus headache, post-operative pain, visceral pain, neuralgia and fibromyalgia;
the cosmetic use is selected from wrinkle removal or prevention, facial slimming, scar repair, improvement or removal of pimples or acne, eyebrow shape modification, mouth corner lifting, body contouring, body odor removal, hair growth and hair retention.
